# EUROPEAN PATENT SPECIFICATION

(11) **EP 1 521 835 B1**
(45) Date of publication and mention of the grant of the patent: **10.03.2010**
(21) Application number: 03762598.5
(22) Date of filing: 03.07.2003
(51) Int. Cl.: C12N 15/54, C12N 15/60, C12N 15/82, C12N 5/10, A01H 5/00

(54) **USE OF AHAS MUTANT GENES AS SELECTION MARKER IN POTATO TRANSFORMATION**
VERWENDUNG VON MUTIERTEN AHAS GENEN ALS SELEKTIONSMARKER BEI DER KARTOFFELTRANSFORMATION
UTILISATION DE GENES MUTANTS AHAS COMME MARQUEURS DE SELECTION DANS LA TRANSFORMATION DES POMMES DE TERRE

(30) Priority: 09.07.2002 EP 02015247
(43) Date of publication of application: 13.04.2005
(60) Divisional application: 10152131.8
(73) Proprietor: BASF Plant Science GmbH, 67056 Ludwigshafen (DE)
(72) Inventor: ANDERSSON, Mariette, S-22229 Lund (SE); TRIFONOVA, Adelina, S-24438 Kävlinge (SE); HOFVANDER, Per, S-23941 Falsterbo (SE)
(74) Representative: Bieberbach, Andreas
(86) International application number: PCT/EP2003/007085
(87) International publication number: WO 2004/005516

(56) References cited:
- WO-A-01/82685
- WO-A-92/11375
- WO-A-92/11376
- WO-A-96/33270
- US-A- 5 767 366
- STOROZHENKO S V ET AL: "Isolation of the transgenic potato plants expressing the mutant gene coding for acetolactate synthase from Arabidopsis thaliana." BIOPOLIMERY I KLETKA, vol. 10, no. 1, 1994, pages 67-71, XP002220128 ISSN: 0233-7657
- LIBIAKOVA G ET AL: "Efficacy of an intron-containing kanamycin resistance gene as a selectable marker in plant transformation." PLANT CELL REPORTS, vol. 20, no. 7, October 2001 (2001-10), pages 610-615, XP002220129 ISSN: 0721-7714 cited in the application

## Description

The present invention relates to an improved, using a mutated acetohydroxy acid synthase (AHAS) gene, for production of transgenic potato lines. Genetic insertion of mutated AHAS genes can give plants tolerance to a number of herbicide compounds. This invention yields a higher transformation efficiency and a reduced escape rate than previously described method for transformation for patato plants.

The invention relates to a method for the generation of stably transformed fertile plants of the genus *Solanum*, which comprises the following steps:
(a) growing the plant to be transformed, and obtaining the suitable explant,
(b) transferring DNA sequences into plant cells,
(c) selecting transformed plant cells and
(d) regenerating transgenic fertile plants.

Moreover, the method comprises the possibility of transferring homologous or heterologous DNA sequences into the plant to be transformed or explants thereof, for example by *Agrobacterium tumefaciens*.

Moreover, the method comprises the possibility of using leaves as explants.

Acetohydroxyacid synthase (EC 4.1.3.18; AHAS; acetolactate sythase) is an enzyme catalysing, in two parallel pathways, the first step of the synthesis of the branched-chain aminoacids valin, leucin and isoleucin. In the vain and leucin biosynthesis, AHAS is catalysing the production of acetolactate by condensation of two pyruvate molecules. While in the isoleucin biosynthesis AHAS condensates one pyruvate molecule with one 2-oxobutyrat molecule to form acetohydroxybutyrat (Umbarger, 1975). Sequence comparison of the AHAS gene in higher plants shows high conservation in at least 10 regions. These regions are probably of great importance for the AHAS function. In tobacco two unlinked genes named SuRA and SuRB code for the AHAS enzyme catalytic subunit. This is not the case in *Arabidopsis thaliana* where only one gene is coding for the enzyme.

AHAS is the target enzyme of several classes of herbicides including sulphonylureas (Ray, 1984), imidazolinones (Shaner et al, 1984), triazolopyrimidines (Subrimanian et al, 1989) and pyrimidinyl oxybenzoat (Hawkes et al, 1989). The herbicides prevent branched amino acids to be synthesised by the plant and may lead to plant death. However, mutations in the AHAS genes can result in higher tolerance to these herbicides (US 5,013,659) because of reduced affinity between enzyme and the herbicide.

In plants mutations conferring resistance to herbicides occur as a result of exposure to the compound repeatedly as it was reported for *Arabidopsis thaliana*. Once the mutated genes are isolated they can be used to genetically engineer plants for improved tolerance to the herbicides. For example mutations in the *Arabidopsis thaliana* ALAS gene have been produced and successfully confer resistance to imidazolinones as described in WO 00/26390, US 5,767,366 and US 6,225,105. Mutations in a corn AHAS gene confer imidazolinone resistance to monocot plants as described in EP 0 525 384.

The mutated AHAS genes can be used for production of herbicide resistant plants, yielding field resistance to a specific herbicide or can be used as a selection marker for genetic engineering of plants.

When genetic material is introduced into a population of plant cells, only a manor part of the cells are successfully transformed. For the production of novel genetically modified plants a selection system is transformed together with the trait genes providing the transformed cells with a selective growth advantage. This construct allows to select transformed from non-transformed cells by adding a compound favoring the regeneration of transformed shoots.

A number of selection systems have been developed; most of them are based on the use of herbicide or antibiotic resistance genes. For many plant species, several of the used selectable marker genes yield a low transformation efficiency and many non-transgenic escapes. Thus more efficient selection systems are required.

The use of antibiotic and herbicide resistance is called negative selection due to that the non-transformed cells are greatly retarded in growth or even killed. Selectable marker genes which can be used are for example the bialaphos resistance gene (bar) and the kanamycin or G418 resistance gene (NPTII) . By growing the tissue on media containing for example kanamycin, in theory only the transformed cells will divide and take part in morphogenesis. The NPTII gene encodes a neomycin phosphotransferase, which reduces the inhibitory action of kanamycin, neomycin, G418 and paromomycin by a phosphorylation reaction.

In contrast to negative selection research has also been done on positive selection, based on giving the transformed cells a metabolic advantage while the non-transformed cells are starving with a concomitant slow reduction in viability. An example is the use of the mannose or xylose based selection system as described e.g. in US 5,767,378.

*Solanum tuberosum* is one of the major target crops for genetic engineering. Main traits for potato are tuber quality, nutritional composition, starch quality, starch yield, insect and virus resistance. One trait with high market potential is the production of high amylopectin starch. The high amylopectin starch has an improved performance in the adhesive and paper industry compared to ordinary starch. In the paper production it will be used as a binder and for coating with printing quality better than latex used today.

Solanum tuberosum is a tetraploid plant with a high level of genetic heterozygosity. Conventional breeding of potato is therefore complicated because of segregation of important characteristics. Genetic engineering has during the last decade been an alternative to conventional breeding when it comes to improving potato varieties. A single trait or a combination of traits are more efficiently introduced by transformation than by using conventional breeding. Due to the fact that genetically modified potato plants can efficiently be vegetatively propagated the trait is fixed in the commercial line without the normal subsequent breeding.

Agrobacterium tumefaciens mediated transformation has successfully been achieved in potato since 1986. Almost exclusively the nptII gene has been used as a selection marker in potato.

The highest documented efficiency using nptII as selection marker in potato transformation is described by Libiakova, G. et al. in 2001. They have produced 120 leaf discs. Of those 81% produced one or more shoots. 90% of the shoots were confirmed transformants and showed resistance to kanamycin. This is equivalent to a regeneration efficiency of 81%, an escape rate of 10% and yields a transformation efficiency of 73%.

It is an object of the present invention to provide an efficient method for transformation of potato plants with a low escape rate and a high transformation efficiency.

Leaf segments are the explants of choice used in Agrobacterium mediated potato transformation. Transgenic plants are regenerated via direct or indirect organogenesis under selective conditions. Cytokinines as growth hormones are mainly involved in provoking cell division and adventitious bud formation in in vitro potato leaf segments. When Imazamox - (RS)-2-(4-isopropyl-4-methyl-5-oxo-2-imidazolin-2-yl)-5-methoxymethylnicotinic acid, an imidazolinone type herbicide - is applied in low concentrations it was surprisingly found that it possesses a cytokinins-line effect in tissue culture, increasing plant cell growth. At higher concentrations the substance is acting as an herbicide. The invention shows that Imazamox can efficiently be used as a selective agent in the recovery of transgenic potato plants and as a cytokinine-like substance it is supporting regeneration during transformation. This phenomenon classifies Imazamox as a unique selection agent that is combining both lethality for non-transgenic cells and improved growth for transgenic cells.

The present invention is describing a new selection system for efficient recovery of transgenic plants. The invention is also describing an innovative selection system that is acting as restriction for untransformed cells and support for transformed ones. Finally the invention is presenting an efficient system for recovery of transgenic potato plants and yields only a very small escape rate.

The present invention relates to the use of a mutated AHAS gene conferring imidazoline type herbicide resistance resulting in a highly efficient selection system for production of transgenic potato lines.

The invention provides a key advantage by minimizing the escapes to almost zero of all shoots regenerated without interfering with the high number of shoots regenerated per explant.

Another advantage is that the method does not comprise a gene conferring resistance to an antibiotic.

For example a mutated gene coding for a functional AHAS enzyme with tolerance to the imadazolinone type herbicides as described in Sathasiavan, K. et al, 1991 is used, see SEQ-ID No. 1. The mutated AHAS gene is used for insertion as a selection marker together with any other homologous or heterologous gene into the potato genome for production of transgenic potato plants. The AHAS enzyme naturally exists in higher plants and the use of a modified plant AHAS gene as selection marker conferring resistance to AHAS inhibiting herbicides does not result in the addition of a new biosynthetic function.

Furthermore
(i) any plant transformation method for insertion of the gene constructs can be used;
(ii) any potato variety can be transformed.

A mutated AHAS gene with mutation S653N as described in SEQ-ID No. 1 and US 5,767,366 was cloned in a vector with the reporter gene beta-gluauronidase (GUS) (Jefferson, R.A. et al., 1987 ) and transformed to potato cells, see example, 1 to 4. The GUS activity is tested in the transgenic lines produced and results in a transformation frequency of 93 to 100% of all transgenic shoots analysed. The transformation method produces a very high amount of shoots per explants, 3 to 5 independent transgenic shoots dependent on the variety used. With a calculation on transformation efficiency per explants the number can be as high as 500%. This is the highest transformation frequency of potato published. The mutated AHAS gene has also been used in combination with a *gbss* antisense gene for production of high amylopectin potato lines, see example 12. The AHAS selection system yielded in this case the same high transformation efficiency, which establishes the use of the AHAS gene as a selection marker also for commercially important traits.

The highest efficiency of *nptII* as a selection marker is 74% (see example 10) of totally analyzed shoots yielding a transformation efficiency of 370% at the maximum. Another selection system that can be used for potato transformation is the Mannose or xylose based selection system. Haldrup et al. (1998) have used the xylose isomerase gene in potato transformation, which yielded a transformation efficiency of 29% based on number of explants producing GUS positive shoots. High percentage of shoots positively transformed has been published for sugar beet and tobacco when using a very high mannose concentration, however the high concentration of the selective agent inhibit the number of shoots per explant distinctively (Joersbo et al., 1998).

The process furthermore comprises the possibility that compounds with herbicidal activity inhibiting the AHAS synthase can be employed for the selection of transgenic potato plants by using different combination of growth regulators as for example auxins, cytokinines and/or auxin or cytokinines conjugates for the regeneration into intact transgenic plants.

The method is suitable for transforming the species Solanum tuberosum.

The invention also relates to Transgenic fertile *Solanum* plants themselves which have been generated by the method described and to their transgenic seeds.

The method according to the invention allows for the first time to transform plant cells of the genus *Solanum* with a transformation efficiency of more than 93% to be regenerated into transgenic plants as shown in example 9.

The method for the generation of stably transformed *Solanum* plants is composed of the following steps: (a) growing the plant to be transformed and obtaining the suitable explant, (b) transferring DNA sequences into plant cells, (c) selecting transformed plant cells with compounds which inhibit in non transformed plant cells the AHAS synthase and (d) regenerating these transformed plant cells into fertile transgenic plants.

A variety of methods is currently available for the transformation. The most frequently employed method for transforming dicotyledonous plants is the *Agrobacterium*-*tumefaciens*-mediated gene transfer. This method exploits the natural ability of the soil bacterium to intestate genetic material into the plant genome. Other suitable methods are for example protoplast transformation by polyethylene-glycol-induced DNA uptake, electroporation, sonication or microinjection, the transformation of intact cells or tissue by micro- or macroinjection into tissue or embryos, tissue electroporation, incubation of dry embryos in DNA-containing solution, vacuum infiltration of seeds and the biolistic gene transfer.

The use of *Agrobacterium tumefaciens* for the transformation of plants using tissue culture explants has been described by Horsch et al., Science 228(1985), 1229-1231; Fraley et al., Proc. Natl. Acad. Sci. USA 80(1983), 4803-4807 and Bevans et al., Nature 304(1983), 184-187. Many strains of *Agrobacterium tumefaciens* are capable of transferring genetic material into *Solanum* species such as for example the strains EHA 101, EHA105, LBA4404 and C58C1 with various disarmed Ti-plasmids.

The agrobacterial strain employed for the transformation of potatoes contains in addition to its disarmed Ti-plasmid a binary plasmid with the T-DNA to be transferred which contains a gene for selecting the transformed cells and the gene to be transferred. Both genes must be equipped with transcriptional and translational initiation and termination signals. The binary plasmid can be transferred into the agrobacterial strain for example by electroporation or other transformation, methods, see Mozo & Hooykaas, Plant Mol. Biol. 16(1991), 917-918. Coculture of the plant explants with the agrobacterial strain takes two to three days.

Foreign genes can be expressed in a constitutive, inducible (by biotic and abiotic factors), tissue-specific or development-specific way. A relatively constitutive expression is achieved for example in plants by the cauliflower mosaic virus 35S promoter. This expression has been described by Shewmaker et al., Virology 140, 281-288 (1985) and Gardner et al., Plant Mol. Biol. 6, 221-228(1986).

Both the direct and the indirect gene transfer are suitable transformation methods. The *Agrobacterium*-mediated transformation using a wide range of starting explants such as for example cotyledons, leaves, hypocotyls, shoots, roots, callus, mature and immature seeds or floral tissue can successfully be employed. The gene transfer can be effected both by simple couultivation/incubation or wetting with the agrobacterial strain and by a supporting vacuum infiltration of the explants with the bacterial culture in question. The use of feeder cultures as aids may be advantageous in the process. Each agrobacterial strain which contains a Ti- or Ri-plasmid with the genetic information required for the transfer is suitable as vector for the transformation. Suitable agrobacterial strains are for example EHA101[pEHA101], EHA105[pEHM05], LBA4404[pAL4404], C58C1[pMP90] and C58C1[pGV2260]. Viral vectors also seem to be suitable for the transformation of *Solanum*. Other methods for transferring genetic material into *Solanum* are for example the polyethylene glycol (PEG)-mediated protoplast transformation, the electroporation, the sonication or microinjection and the transformation of intact cells or tissue by micro- or macroinjection into tissue or embryos, tissue electroporation, incubation of dry embryos in DNA-containing solution, the vacuum infiltration of seeds, see also Eds. Galun, E. and Breiman, A. In: Transgenic Plants, Imperial College Press, 1997. The use of the particle gun for bombarding a wide range of explants such as for example leaves and callus, is also possible.

All gene transfer vectors which contain the border sequences necessary for transferring the T-DNA (left and right border, LB and RB respectively), carry one or more selectable marker or reporter genes under the control of suitable promoters and terminator and/or contain further useful genes or target genes, also under the control of suitable transcriptional and translational regulatory units, can be employed for the *Agrobacteria-*mediated transformation. The transfer of DNA sequences into the *Solanum* plant to be transformed or explants thereof is preferably done by *Agrobacterium*-*tumefaciens*-mediated gene transfer.

The regeneration medium according to the invention furthermore comprises growth regulators such as auxins and/or auxin conjugates, and cytokinins and/or cytokinin conjugates. Suitable auxins are both natural and synthetic auxins. The natural auxin is indoleacetic acid (IAA), examples of synthetic auxins are 3-indolebutyric acid (IBA), 1-naphthylacetic acid (NAA) and the herbicide 2,4-dichlorophenoxyacetic acid (2,4-D). Other herbicides which act as auxins are also feasible as growth regulators. Auxin conjugates are compounds of auxin (IAA) with aspartic acid, glucose, myoinositol and others. In the case of the cytokinins, again, natural cytokinins such as, for example, zeatin and synthetic components, such as 6-benzylaminopurine (BAP) and 6-furfurylaminopurine (kinetin) may also be employed. Zeatin riboside is frequently employed as cytokinin conjugate. The auxins and the cytokinins can be employed in each case as individual components, but also as auxin or cytokinin mixtures. The concentration, of the individual growth regulators is 0.05 to 10 mg/l, preferably 0.1 to 5 mg/l.

The transformation method according to the invention can be applied, inter alia, to Solanaceae species as Solanum tuberosum (potato), Lycopersicon esculentum (tomato) and pepper.

The mutant AHAS gene of the present invention confers resistance to imidazolinone herbicides Types of herbicides to which resistance is conferred are described for example in US Patent Nos.: 4,188,487; 4,201,565; 4,221,586; 4,297,128; 4,554,013; 4,608,079; 4,638,068; 4,747,301; 4,650,S14; 4,698,092; 4,701,208; 4,709;036; 4,752;323; 4,772,311 and 4,798,619.

It will be understood by those working in the field that the nucleic acid sequence depicted in SEQ-ID No. 1 is not the only sequence which can be used to confer imidazolinone-specific resistance. Also contemplated are those nucleic acid sequences which encode an identical protein but which, because of the degeneracy of the genetic code, possess a different nucleotide sequence. The invention also encompasses genes encoding AHAS sequences in which the above-mentioned mutation is present, but which also encode one or more silent amino acid changes in positions of the molecule not relevant for resistance to herbicides or to the catalytic function. Also contemplated are gene sequences from other imidazolinone resistant monocot or dicot plants which have a mutation in the corresponding region of the sequence.

For example, alterations in the gene sequence which results in the production of a chemically equivalent amino acid at a given site are contemplated; thus, a codon for the amino acid alanine, a hydrophobic amino acid, can readily be substituted by a codon encoding another hydrophobic residue, such as glycine, or may be substituted with a more hydrophobic residue such as valine, leucine or isoleucine. Similarly, changes which result in a substitution of a negatively charged residue for another, such as aspartic acid for glutamic acid, or one positively charged residue for another, such as lycine for arginine, can also be expected to produced a biologically equivalent product.

For expression of the mutated AHAS gene conferring herbicide resistance in potato the following promoters can be used:
- the nos promoter
- the AHAS promoter as described in US 6,025,541;

The invention can especially be carried out by using the AHAS promoter, the AHAS resistance gene S653N as described in US 5,767,366 and the AHAS terminator from Arabidopsis thaliana cut from pAC321 as described in example 3.

The Arabidopsis AHAS gene (S653N) used for transformation and selection contains most of the common restriction sites for cloning such as HindIII, BamHI, EcoRI, SstI, BglII and EcoRV. Alter-action of the Molecular composition of the AHAS gene can be performed to eliminate restriction sites without altering the amino acid sequence of the resulting protein. Special care must be taken not to alter the codon usage profile from the one found in Arabidopsis, which could lead to reduced translation efficiency.

The designed gene can be made synthetically and accommodates suitable restriction sites at 5' and 3' ends for cloning.

Comparative studies have to be performed in potato in order to confirm that the synthetically made gene does not differ from the original mutated AHAS gene in conferring herbicide resistance.

For selection of transgenic potato plants chemical compounds inhibiting the AHAS enzyme can be used. Usefull compounds are the imidazoline type herbicides. Especially useful compounds are selected from the group consisting of imazethapyr (Pursuit^{™}), imazamox (Raptor^{™}), imazamethabenz (Assert^{™}), imazapyr (Aresenal^{™}), imazapic (cadre^{™}) and imazaquinon (Scepter^{™}).

For selection of transgenic plants chemical compounds as described in the review article by Duggleby, R.G. and Pang, S.S. in Journal of Biochemistry and Molecular Biology 33(1), 1-36 (2000) can be used.

The mutated AHAS gene as described in SEQ-ID No. 1 has been established as a selection marker in five different potato varieties, see table 2 and 3, and can thus be used for transformation of potato varieties in general. Two different selection pressures, using 0.3 and 0.5 µM Imazamox, have been tested, see table 2. For selection on 0.3 µM Imazamox the transformation efficiency is ranging from 7 to 76 % depending on the variety used. When increasing the selection to 0.5 µM Imazamox the transformation rate is increasing to 93 to 100% respectively. The high transformation efficiency at 0.5 µM Imazamox is independent from the variety used. The fifth variety Seresta has as can be seen in table 3 and 4 a transformation efficiency of 98 to 100% at 0.5 µM Imazamox.

The invention furthermore relates to the use of a plant expression vector comprising SEQ-ID No. 1 wherein the heterologeous DNA sequence encodes a peptide, protein, antisense-, sense-RNA, viral RNA or ribozyme.

The specification furthermore discloses the use of a plant expression vector comprising SEQ-ID No. 1 wherein the heterologeous DNA sequence contains information that e.g. causes changes in the carbohydrate concentration and the carbohydrate composition of regenerated potato plants.

The specification furthermore discloses the use of a DNA sequence SEQ-ID No. 1 or a DNA sequence comprising a nucleotide sequence which hybridizes to a complementary strand of the nucleotide SEQ-ID No. 1 or a DNA sequence comprising a nucleotide sequence which is degenerated to the nucleotide Sequence SEQ-ID No. 1 or a DNA sequence being a derivative, analogue or fragment of a nucleotide sequence encoding a protein possessing AHA synthase activity and conferring resistance to AHA synthase inhibitors.

The experiment shown in example 12 with the high amylopectin trait establishes the use of the AHAS resistance gene conferring resistance to herbicides inhibiting the AHAS enzyme as a selection marker also for commercially important traits. The transformation efficiency is 98 and 100% respectively. Analysis of the amylopectin/amylose ratio shows that in the transgenic shoots selected the amylopectin content is higher than in non transgenic control shoots. This is proof that by using the invention as described commercially relevant transgenic potato plants can be generated.

The invention now having been generally described will be more readily understood by reference to the following examples, which are included for the purpose of illustration only, and are not intended to limit scope of the present invention.

### Example 1

### Test for transgenicity - Detection of glucuronidase expression

Microtubers of the regenerated in vitro potato plants were subjected to a qualitative glucuronidase (GUS) enzyme detection by infiltrating cut microtubers for 1 minute in vacuo with the GUS substrate 5-bromo-4-chloro-3-indolyl-β-D-glucuronic acid (X-GlcA) in a 100 mM sodium phosphate buffer, pH 7.0, which contained 10 mM EDTA, 0.1% Triton X100 and 10 mM DTT, and subsequently incubating them for approximately 15 hours at 37°C. Microtubers showed an intense blue coloration, which proves that the reporter gene is expressed in *Solanum.*

### Example 2

### Investigation of selection pressure

Potato leaf segments from in vitro propagated plants of four different potato varieties Prevalent, Producent, Karnico and Desiree were tested for natural tolerance on different concentrations of the imidazolinone herbicide Imazamox. It is important to choose a selection concentration at which the potato leaf tissue is viable long enough to regenerate shoots but have a high enough concentration to prevent the regeneration of untransformed shoots. Doses 0.1, 0.5, 1, 5, 10, 20, 30 and 50 µM Imazamox were tested.

Fully expended potato leaves are diagonally cut in 2 pieces and precultivated on MC-plates for 2 to 3 days at 23 to 24°C, see table 1.

The leaf tissues are transferred on MS300 medium for additional 2 days and cultivated under modest light at 23 to 24°C simulating a co-cultivation step. Subsequently the leaf segments are moved to MS400 plates containing 400mg/l Claforan. Claforan is added in order to suppress growth of *Agrobacterium tumefaciens*. Therefore the regeneration of shoots on different concentrations of Imazamox need to be monitored in presence of Claforan. Selection is taking place within 4 to 5 days when the explants are moved to MS400 medium supplemented with 400mg/l Claforan and Imazamox at above concentrations.

The explants are transferred to fresh selection medium every fortnight.

**Table 1: Media used for potato transformation.**

| MC plates | MS300 |
|---|---|
| | |
| MS300 plates with 1.5-2 ml | X MS-medium |
| liquid MS 100 medium and | 2mg/l NAA (naphtyl acetic acid) |
| covered with one sterile | 1mg/l BAP (6-benzyl amino pyridine) |
| filter paper | 3% (w/v) sucrose |
| | pH 5.2 |

| MS 10 | MS400 |
|---|---|
| | |
| 4.4 g/l MS-medium (Murashige and Skoog) | 4.4 g/l MS-medium |
| 1% (w/v) sucrose | 2mg/l zeatine |
| pH5.8 | 0.01 mg/l NAA (naphtyl acetic acid) |
| | 0.1 mg/l GA3 (giberellic acid) |
| | 10% (w/v) sucrose |
| | 400 mg/l claforan |
| | Imazamox or kanamycin |
| | pH5.8 |

| MS 30 | Microtuber medium |
|---|---|
| | |
| 4.4 g/l MS-medium | 4.4 g/l MS-medium |
| 3% (w/v) sucrose | 2.5 mg/l kinetin |
| pH 5.8 | 0.5 mg/l ABA (abscisic acid) |
| | 8% sucrose |
| | 200 mg/l claforan |

| MS100 | |
|---|---|
| | |
| 4.4 mg/l MS-medium | |
| 30g/l sucrose | |
| 0.5 mg/l thiamin-HCl | |
| 0.5 mg/l pyridoxin-HCl | |
| 1 mg/l nicotinacid | |
| 0.5 mg/l kinetin | |
| 29.8 mg/l ferrous sulfate hepta hydrate | |
| 1 mg/l 2,4-Dichlorophenoxyacetic acid | |
| 2 g/l caseinhydrolysate | |
| pH 5.2 | |

### Example 3

### Construction of binary vectors pAHASGUS, pAP1 and pAP2

For all constructs the same AHAS mutant gene with mutation S653N originating from *Arabidopsis thaliana* was used (Sathasivan, K. et al., 1991).

### pAHASGUS

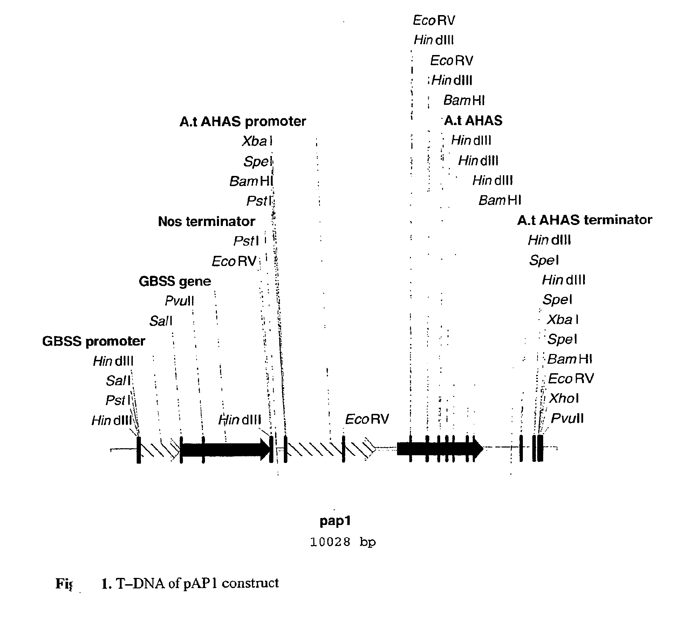

AHAS gene with mutation S653N as described in SEQ ID No. 1, originating from *Arabidopsis thaliana,* was used together with the nos promoter (Herrera, L. et al., 1983) and the OCS terminator (Wesley S. V. et al., 2001) in the binary vector pGPTVkan (Becker, D. et al., 1992 ). The pBIN19 based pGPTVkan was digested with ApaI and BamHI discarding the *nptII* gene. The remaining 12600 bp contained from right border the nos terminator, the uidA gene (also known as GUS), the cloning cassette, the nos promoter and the pAg7 terminator. The AHAS gene with mutation S653N and the OCS terminator was ligated to the digested pGPTVkan and named pABA1, see figure 1.

For expression of the GUS gene a tuber specific gbss promoter 988bp (WO 92/11376) was cloned at the SmaI site of pABA1.

The resulting pAHASGUS construct was used for *Agrobacterium tumefaciens* transformation of potato, where regenerated plantlets were analysed for GUS expression to determine the transformation efficiency.

### pAP1

AHAS gene with mutation S653N, driven by the *Arabidopsis thaliana* AHAS promoter as described in US 5,750,866, was used as a selection gene for co-transformation with a granule-bound starch synthase (gbss) antisense gene (EP-A 0 563 189).

A 293 bp nos terminator was cut out from pHAxwO and ligated to pBluescript with EcoRI. The 2952 bp gbss promotor and gbss gene in antisense direction (EP-A 0 563 189) was cut from pHAxwO and ligated in front of the nos terminator with HindIII resulting in pMJ2. The gbss complex was cut out from pMJ2 with XbaI-XhoI and ligated to pSUN1 (WO 02/00900) cut with XbaI-SalI resulting in the construct named pMJ3.

pAC321 was cut with XbaI which yielded a fragment of 5717 bp containing AHAS promoter, AHAS gene and AHAS terminator all originating from *Arabidopsis thaliana* as described in SEQ-ID No. 1.

SEQ-ID No.1 contains the following elements:
1-2483 At AHAS promoter
2484-4496 At AHAS gene
4497-5717 At AHAS terminator

The pAC321 fragment was ligated to pMJ3 opened with XbaI.

The resulting pAP1 (figure 1) construct was used for *Agrobacterium tumefaciens* transformation of potato, which were analysed for high amylopectin starch quality.

### pAP2

AHAS gene with mutation S653N, driven by the nos promoter (US 6,174,724), was used as a selection marker for co-transformation with a gbss antisense gene (WO 92/11376).

A nos promotor of 600 bp was cut out from pGPTVKan (Becker et al., 1992) with HindIII, blunted and cut with BglII. The fragment was ligated to pBluescript (Stratagene) cut with SpeI, blunted and cut with BamHI. The construct was named pPnos2.

A 2986 bp fragment of pACGH101 containing the AHAS gene with mutation S653N as described in SEQ ID No. 1 and OCS terminator was cut out with PstI and PvuII. The AHAS gene originates from *Arabidopsis thaliana* see pAP1. The pACGH101 fragment was ligated to pPnos2 cut with PstI and EcoRV and named pPnoas.

The 3406 bp AHAS complex was cut out from pPnoas with XbaI-PvuII and ligated to pMJ3 (see pAP1) cut open with XbaI.

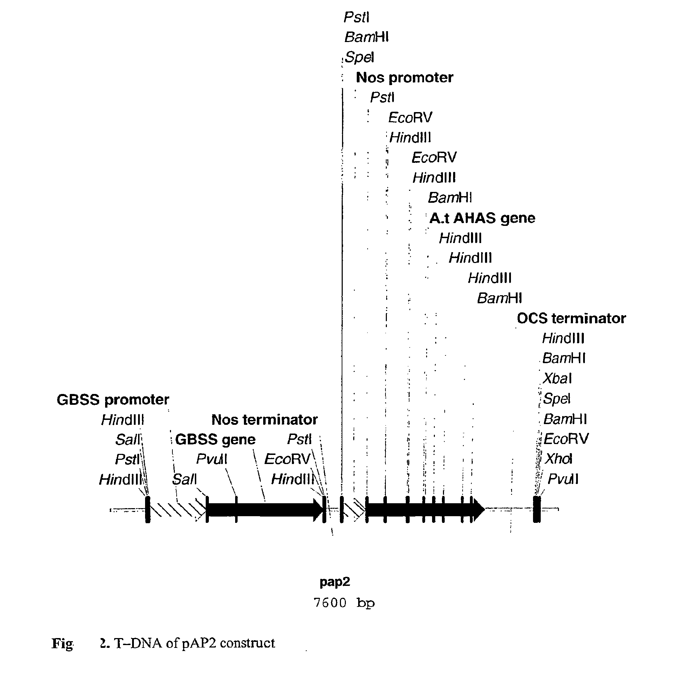

The resulting pAP2 (figure 2) construct was used for *Agrobacterium tumefaciens* transformation of potato, which were analysed for high amylopectin starch quality.

### Example 4

### Transformation method

Fully expanded leaves from in vitro propagated potato plants are diagonally cut in 2 pieces and precultivated on MC-plates for 2 to 3 days at 23 to 24°C.

Agrobacterium tumefaciens containing pAHASGUS is grown in YEB medium containing 25 µg/ml kanamycin and 100 µg rifampicin and grown over night on constant shaking (200 rpm) at 28°C.

The Agrobacterium culture is prepared for infection by dilution 1:20 with MS10 medium. The leaf explants are infected for 8-10 min in the bacterial solution and afterwards drained on filter paper for 5 to 20 seconds. The leaf segments are placed on the MS300 plates for 2 days co-cultivation under modest light at 23 to 24°C. At the end of co-cultivation the leaf segments are moved to M400 plates containing 400 mg/l Claforan to suppress bacterial growth. After 4 to 5 days the explants are moved to selection medium MS400 supplemented with 400 mg/l Claforan and Imazamox at the previously detected concentrations of 0.3 or 0.5 µM.

Leaf segments are transferred to fresh MS 400 selection medium every fortnight. The regenerated putative transgenic shoots are collected and cultivated on MS30 plates with 200mg/l Claforan aiming at shoot elongation.

When the shoots are 3 to 5 cm long, 1 to 2 cm are cut off and grown on microtuber medium in the dark at 25°C. After 2 to 5 weeks microtubers are produced. Putative transgenic plants are analysed for GUS expression in microtubers to determine the transformation efficiency.

### Example 5

GUS expression analysis for determination of the transformation efficiency

A thin slice from each microtuber was placed in a microtiter plate. The slices were incubated with 1 mM X-gluc in 50 mM NaH₂PO₄ pH 7.2 at 37°C for approximately 1 hour. A very distinct blue colour appeared on tuber slices if a successful integration and expression of the GUS gene was achieved.

### Example 6

Transformation efficiency determination of pAP1 and pAP2 by PCR analysis

Transgenic shoots were distinguished from non-transgenic shoots - for example escapes - by using PCR. DNA was extracted according to DNeasy 96 Plant protocol (Qiagen, Germany). In a 96 well microtiter plate, 10 to 15 mg leaf tissue was added to each well together with a 5 mm steel ball each well then representing one individual shoot. The plates were frozen in N₂(1) before homogenisation. The homogenisation was done at 30 Hz in a Mixer-mill 300 for 1 min. The DNA was at the end of the extraction protocol eluted in 75 ml H₂O.

Specific primers were used to amplify a fragment of the mutant S653N AHAS gene. Successful integration of the mutant AHAS gene results in a fragment of 509 bp upon PCR amplification using the specific primers.

| | | |
|---|---|---|
| Forward primer: | AHAS1_frw: | AACAACAACATCTTCTTCGATC |
| Reverse primer: | AHAS1_rev: | TAACGAGATTTGTAGCTCCG |

The PCR reactions were done with the extracted DNA setup and run as follows:

### Reaction:

| | |
|---|---|
| 10x PCR Mix | 2,0 µl |
| Primer frw (25 µM) | 0,4 µl |
| Primer rev (25 µM) | 0,4 µl |
| dNTPs (10 mM) | 0,4 µl |
| RedTAQ (Sigma) | 1,0 µl |
| Templat (∼20 ng/µl) | 4,0 µl |
| H₂O | 11,8 µl |

### PCR program:

| | |
|---|---|
| 94°C | 30 s |
| 59°C | 30 s x 29 cycles |
| 72°C | 30 s |
| 72°C | 7 min |
| 8°C | Hold |

Negative and positive control was included in all runs.
The reactions were analysed on 1.5% agarose gels.

### Example 7

### Starch quality analysis of amylopectin/amylose ratio

The screening for an altered amylopectin/amylose ratio was performed on transgenic lines selected for high quality integration of the intended genetic insert. The screening was done by staining with iodine (Lugol's solution: (6.7 g/l KI + 3.3 g/l I₂) and (glycerol); ratio 1:1). Iodine stains starch containing amylose blue and starch exclusively containing amylopectin red-brown. A microtuber was crushed and a few drops of Lugol's solution were added. The starch amylopectin quality was analysed under the microscope according to the staining colour of the starch.

### Example 8

Leaf tissues were tested for the use of the AHAS resistance gene contained in SEQ ID No. 1 and for the selection of transgenic potato plant cells as described above. Four different potato cultivars Prevalent, Producent, Karnico and Desiree were cultivated on different concentrations of Imazamox, an imidazolinones type herbicides. It is essential to use the proper concentration for selection to yield an acceptable number of shoots per explant without high escape rate. The lethality of various Imazamox concentrations on regeneration was evaluated.

Lethal dose for regeneration was detected within 0.1 to 1 µM ranges of concentrations. Slight variation in lethality was detected between varieties. No regeneration occurs when 0.5 µM Imazamox was included in the medium. The concentrations 0.3 µM and 0.5 µM are selected for transformation experiments providing a lethality of 80 and 100% in regeneration.

### Example 9

Transformation of four different potato varieties (Prevalent, Producent, Kuras and Desiree) was performed with pAHASGUS as described above. For selection a concentration of 0.3 or 0.5 µM Imazamox was used. Each shoot was analysed according to GUS activity and the number of GUS positive shoots in total is listed in table 2. A distinct blue colour appears on the microtuber slices if a successful integration of the GUS gene was achieved. From each explant 3 to 5 independent shoots were regenerated depending on the variety used. In all experiments the efficiency of positive shoots per explants is stated and the figures in table 2 have to be multiplied 3 to 5 times, yielding a transformation efficiency of up to 500%. Compared with selection systems used previously in potato plants the big advantage of the invention described is that together with a very high efficiency the number of shoots per explant is very high. For all four varieties transformed an extremely high transformation efficiency of up to 93% to 100% of all shoots regenerated was achieved at an Imazamox concentration of 0.5 µM. The experiment using 0.3 µM of Imazamox for selection shows how important the Imazamox concentration is for an efficient transformation rate. This can be seen from the results for variety Kuras in which a transformation efficiency of 7% at 0.3 µM Imazamox is increased to 93% at the 0.5 µM level.

**Table 2: Transformation efficiency of four different varieties with 0.3 and 0.5 µM Imazamox used as the selective agent.**

| **Variety** | **Construct** | **Selection Imazamox** | **Selection Imazamox** | **Number analysed** | **Number of positive** | **% positive** |
|---|---|---|---|---|---|---|
| | | **(µM**) | **(mg/l)** | | | |
| Desiree | pAHASGUS | 0.3 µM | 0.0915mg/l | 19 | 12 | 63 |
| Prevalent | pAHASGUS | 0.3 µM | 0.0915mg/l | 26 | 16 | 62 |
| Producent | pAHASGUS | 0.3 µM | 0.0915mg/l | 25 | 19 | 76 |
| Kuras | pAHASGUS | 0.3 µM | 0.0915mg/l | 58 | 4 | 7 |
| | | | | | | |
| Prevalent | pAHASGUS | 0.5 µM | 0.1525mg/l | 23 | 23 | 100 |
| Producent | pAHASGUS | 0.5 µM | 0.1525mg/l | 40 | 38 | 95 |
| Kuras | pAHASGUS | 0.5 µM | 0.1525mg/l | 33 | 32 | 97 |
| Desiree | pAHASGUS | 0.5 µM | 0.1525mg/l | 29 | 27 | 93 |

### Example 10

In a similar experiment compared to example 9 the *nptII* gene was used as selection marker being controlled by the same promoter nos as in pAHASGUS. The shoots are selected on 50 mg/l kanamycin, which is a standard kanamycin concentration used for potato transformation as described in Ooms, G. et al., 1987 and Tavazza, R. et al., 1988. The transformation frequency was much lower compared to when using a mutated AHAS gene e.g. the S653N gene as selection marker. The highest transformation efficiency based on analysis of the same GUS gene driven by the same GBSS promoter resulted in 74% transformed shoots regenerated.

### Example 11

The successful use of a mutated AHAS gene as a selection marker has been demonstrated using the promoter from the wild-type *Arabidopsis thaliana* AHAS gene (pAP1 construct) as well as a recombinant nos promoter (pAP2 construct) in the potato variety Seresta, see table 3. This shows that any promoter with sufficient expression can be used in connection with the mutated AHAS gene and thus the invention could be used with different promoters as regulatory elements for the mutated AHAS gene successfully yielding selection of transgenic shoots on selective medium. The AHAS gene can be driven by different promoters such as e.g. the AHAS-, nos-, 35S-, Rubisco- or vATPase - promoter.

**Table 3: Transformation efficiency of the mutated AHAS gene driven by two different promoters: Arabidopsis thaliana (A.t.) AHAS promoter and the nos promoter.**

| **Variety** | **Construct** | **Promoter** | **Number shoots analysed** | **Number shoots positive** | **% positive** |
|---|---|---|---|---|---|
| Seresta | pAP1 | A.t AHAS | 50 | 49 | 98 |
| Seresta | pAP2 | nos | 120 | 117 | 98 |

### Example 12

The mutated AHAS gene conferring resistance to Imazamox was used as a selection marker for the transformation of a commercially important trait to *Solanum tuberosum* (variety Seresta) resulting in potato lines producing amylopectine type starch. The starch component amylose is synthesized by granule-bound starch synthase (GBSS). Inhibition of the gene coding for GBSS directs starch production completely to amylopectin. An antisense gene fragment inhibiting the expression of gbss as described in EP-A 0 563 189 was co-transformed with the mutated AHAS gene conferring resistance to Imazamox.

The gbss promoter was used to drive the gbss antisense gene fragment. The Nos promoter or the AHAS promoter was used to drive the AHAS selection gene and resulted in a total transformation efficiency per shoots analysed of 98 to 100%, see table 4.

For restriction map of the T-DNA of the pAP1 and pAP2 constructs used, see figure 1 and 2. There were 3 to 5 independent shoots produced per explant yielding a transformation efficiency of 280 to 480% or up to 500% per explant. There were no differences in transformation efficiency between the different promoters and *Solanum tuberosum* varieties used.

**Table 4: pAP1 and pAP2 transgenic lines analysed for transformation efficiency and high amylopectin quality.**

| **Experiment** | **Variety** | **Construct** | **Number shoots analysed** | **Number shoots positive** | **% positive** | **high amylopectin content** |
|---|---|---|---|---|---|---|
| 1 | Seresta | pAP2 | 120 | 117 | 98 | + |
| 2 | Seresta | pAP1 | 50 | 49 | 98 | + |
| 3 | Seresta | pAP2 | 30 | 30 | 100 | + |
| 4 | Seresta | pAP2 | 3 | 3 | 100 | + |

Starch quality analysis was done with microtubers of the positive shoots with high quality integration of the intended genetic insert. The starch was mixed with iodine and the shoots exclusively containing amylopectin stains red-brown compared to the starch containing amylose that stains blue.

### References

Hawkes, T.R., in Prospects for Amino Acid Biosynthesis Inhibitors in crop protection and Pharmaceutical Chemistry, pp131-138, Brittish Crop Protection Council Monograph No 42, Surrey, U.K. (1989)
Herrera-Estrella, L., Depicker, a., Van Montagu, M., Schell, J., Expression of chimaeric genes transferred into plant cells using Ti-plasmid-derived vector, Nature 303:209-213 (1983)
Becker, D., Kemper, E., Schell, J., Masterson, R., New plant binary vectors with selectable markers located proximately to the left T-DNA border, Plant Molecular Biology 20:1195-1197, (1992)
Jefferson, R.A., Kavanagh, T.A., Bevan, M.W., GUS fusions: b-glucuronidase as a sensitive and versatile gene fusion marker in higher plants, EMBO Journal 6: 3901-3907 (1987)
Joersbo, M., Donaldson, I., Kreiberg, J., Guldager Petersen, S., Brunstedt, J., and Okkels, F.T., Analysis of mannose selection used for transformation of sugar beet, Molecular Breeding 4:111-117 (1998)
Libiakova, G., Jorgensen, B., Palmgren, G., Ulskov, P., Johansen, E., Efficacy of an intron containing kanamycin resistance gene as a selectable marker in plant transformation, Plant Cell Rep 20:610-615(2001)
Ooms, G., Burell, M.M., Karp, A., Bevan, M., Hille, J., Genetic transformation in two potato cultivars with T-DNA from disarmed Agrobacterium, Theoretical and Applied Genetics (1987) 73:744-750,
Ray, T.B., Plant Physiology (1984), 75, 827-831
Sathasivan, K., Haughn, G.W., Murai, N., Plant Physiology 97(1991), 1044-1050
Shaner, D.L., Andersson, P.C., Stidham, M.A., Plant Physiology 76 (1984), 545-546
Subrimanian, M.V., Gerwick, B.C., in Biocatalysis in Agricultural Biotechnology, pp277-288, ACS Symposium Series No389, American Chemical Society, Washington DC (1989)
Tavazza, R., Tavazza, M., Ordas, R.J., Ancora, G., Benvenuto, E., Genetic transformation of potato (Solanum tuberosum): An efficient method to obtain transgenic plants, Plant Science, 59 (1988), 175-181
Umbarger, H.E., in Synthesis of Amino Acids and Proteins, (1975) pp1-56, MTP International Review of Science, Butterworth, London.
Wesley S.V., Helliwell C.A., Smith N.A., Wang M.B., Rouse D.T., Liu Q., Gooding P.S., Singh S.P., Abbott D., Stoutjesdijk P.A., Robinson S.P., Gleave A.P., Green A.G., Waterhouse P.M., Construct design for efficient, effective and high-throughput gene silencing in plants, Plant J 2001 Sep;27(6):581-90
Haldrup A, Petersen, GP, Okkels FT (1998) The xylose isomerase gene from Thermoanaerobacterium thermosulfurogenes allows effective selection of transgenic plant cells using D-xylose as the selection agent. Plant Molecular Biology 37:287-296

## Claims

1. A method for transformation of potato plants by transforming potato plant cells with an expression vector comprising
a) a regulatory sequence of an AHAS promoter operably linked to a nucleotide sequence, encoding a protein with the biological activity of an AHA synthase according to SEQ-ID NO.2 and being resistant to the inhibitor (RS)-2-(4-isopropyl-4-methyl-5-oxo-2-imidazolin-2-yl)-5-methoxy-methylnicotinic acid, operably linked to an AHAS terminator
or
b) a regulatory sequence of a nos promoter operably linked to a nucleotide sequence, encoding a protein with the biological activity of an AHA synthase according to SEQ-ID No. 2 and being resistant to the inhibitor (RS)-2-(4-isopropyl-4-methyl-5-oxo-2-imidazolin-2-yl)-5-methoxy-methylnicotinic acid, operably linked to an OCS terminator; and
selecting for AHA synthase inhibitor resistant cells and regenerating them to transgenic plants.

2. A method for transformation according to claim 1, wherein the expression vector comprises
a) a nucleotide sequence according to SEQ-ID NO. 1
b) a nucleotide sequence which hybridizes to a complimentary strand of the nucleotide sequence a) encoding a protein possessing AHA synthase activity according to SEQ-ID NO. 2 and conferring resistance to AHA synthase inhibitors.
c) a nucleotide sequence which is degenerate to the nucleotide sequence of a) and
d) a derivative, analogue or fragment of a nucleotide sequence of a), b) or c) encoding a protein possessing AHA synthase activity according to SEQ-ID NO. 2 and conferring resistance to AHA synthase inhibitors.

3. A method for transformation according to any of claims 1 or 2, wherein for selection an imidazolinone type herbicide is used.

4. A method for transformation according to claim 3, wherein for selection (RS)-2-(4-isopropyl-4-methyl-5-oxo-2-imidazolin-2-yl)-5-methoxymethylnicotinic acid is used.

5. A transgenic potato plant produced by the method of transformation according to any of claims 1 to 4, wherein the plant exhibits an elevated resistance to imidazolinone type herbicides.

6. A transgenic potato plant according to claim 5 wherein the plant exhibits an elevated resistance to (RS)-2-(4-isopropyl-4-methyl-5-oxo-2-imidazolin-2-yl)-5-methoxymethylnicotinic acid.

7. A cell of a transgenic potato plant according to claim 5 or 6.

8. A tuber of a transgenic potato plant according to claim 5 or 6.

9. Use of an expression vector as **characterized in** claim 1 or 2 for selecting transgenic potato plant cells, transgenic potato tissue cultures or transgenic potato plants.

## Patentansprüche

1. Transformationsverfahren für Kartoffelpflanzen **dadurch**, dass man Kartoffelpflanzenzellen mit einem Expressionsvektor, umfassend
a) eine Regulationssequenz für einen AHAS-Promoter in operativer Verknüpfung mit einer Nukleotidsequenz, die für ein Protein mit der biologischen Aktivität einer AHA-Synthase gemäß SEQ ID Nr.: 2 und mit Resistenz gegenüber dem Hemmer (RS)-2-(4-Isopropyl-4-methyl-5-oxo-2-imidazolin-2-yl)-5-methoxymethylnicotinsäure codiert, in operativer Verknüpfung mit einem AHAS-Terminator, oder
b) eine Regulationssequenz eines nos-Promoters in operativer Verknüpfung mit einer Nukleotidsequenz, die für ein Protein mit der biologischen Aktivität einer AHA-Synthase gemäß SEQ ID Nr.: 2 und mit Resistenz gegenüber dem Hemmer (RS)-2-(4-Isopropyl-4-methyl-5-oxo-2-imidazolin-2-yl)-5-methoxymethylnicotinsäure codiert, in operativer Verknüpfung mit einem OCS-Terminator,
transformiert, und auf AHA-Synthasehemmer-resistente Zellen selektiert und diese zu transgenen Pflanzen regeneriert.

2. Transformationsverfahren nach Anspruch 1, wobei der Expressionsvektor Folgendes umfasst:
a) eine Nukleotidsequenz gemäß SEQ ID Nr.: 1,
b) eine Nukleotidsequenz, die mit einem Komplementärstrang der Nukleotidsequenz a) hybridisiert und die für ein Protein mit AHA-Synthaseaktivität gemäß SEQ ID Nr.: 2 codiert und Resistenz gegenüber AHA-Synthasehemmern vermittelt,
c) eine Nukleotidsequenz, die gegenüber der Nukleotidsequenz gemäß a) degeneriert ist, und
d) ein Derivat, Analog oder Fragment einer Nukleotidsequenz gemäß a), b) oder c), das für ein Protein mit AHA-Synthaseaktivität gemäß SEQ ID Nr.: 2 codiert und Resistenz gegenüber AHA-Synthasehemmern vermittelt.

3. Transformationsverfahren nach einem der Ansprüche 1 oder 2, wobei für die Selektion ein Herbizid des Imidazolinontyps verwendet wird.

4. Transformationsverfahren nach Anspruch 3, wobei für die Selektion (RS)-2-(4-Isopropyl-4-methyl-5-oxo-2-imidazolin-2-yl)-5-methoxymethylnicotinsäure verwendet wird.

5. Transgene Kartoffelpflanze, die nach dem Transformationsverfahren nach einem der Ansprüche 1 bis 4 erzeugt wurde, wobei die Pflanze eine erhöhte Resistenz gegenüber Herbiziden des Imidazolinontyps aufweist.

6. Transgene Kartoffelpflanze nach Anspruch 5, wobei die Pflanze eine erhöhte Resistenz gegenüber (RS)-2-(4-Isopropyl-4-methyl-5-oxo-2-imidazolin-2-yl)-5-methoxymethylnicotinsäure aufweist.

7. Zelle einer transgenen Kartoffelpflanze nach Anspruch 5 oder 6.

8. Knolle einer transgenen Kartoffelpflanze nach Anspruch 5 oder 6.

9. Verwendung eines Expressionsvektors wie in Anspruch 1 oder 2 beschrieben für die Selektion von transgenen Kartoffelpflanzenzellen, transgenen Kartoffelgewebekulturen oder transgenen Kartoffelpflanzen.

## Revendications

1. Méthode de transformation de plantes de pomme de terre, par la transformation de cellules de plantes de pomme de terre par un vecteur d'expression comprenant :
a) une séquence régulatrice d'un promoteur AHAS lié de manière opérante à une séquence nucléotidique, codant pour une protéine ayant l'activité biologique d'une AHA synthase selon SEQ ID n°2 et étant résistante à l'inhibiteur, l'acide (RS)-2-(4-isopropyl-4-méthyl-5-oxo-2-imidazolin-2-yl)-5-méthoxyméthylnicotinique, lié de manière opérante à un terminateur AHAS ;
ou
b) une séquence régulatrice d'un promoteur nos lié de manière opérante à une séquence nucléotidique, codant pour une protéine ayant l'activité biologique d'une AHA synthase selon SEQ ID n°2 et étant résistante à l'inhibiteur, l'acide (RS)-2-(4-isopropyl-4-méthyl-5-oxo-2-imidazolin-2-yl)-5-méthoxyméthylnicotinique, lié de manière opérante à un terminateur OCS ; et
la sélection des cellules résistantes à l'inhibiteur de l'AHA synthase et leur régénération en plantes transgéniques.

2. Méthode de transformation selon la revendication 1, dans laquelle le vecteur d'expression comprend :
a) une séquence nucléotidique selon SEQ ID n°1 ;
b) une séquence nucléotidique qui s'hybride à un brin complémentaire de la séquence nucléotidique a) codant pour une protéine possédant une activité d'AHA synthase selon la SEQ ID n°2 et conférant une résistance aux inhibiteurs de l'AHA synthase ;
c) une séquence nucléotidique qui est dégénérée par rapport à la séquence nucléotidique de a) ; et
d) un dérivé, analogue ou fragment d'une séquence nucléotidique de a), b) ou c) codant pour une protéine possédant une activité d'AHA synthase selon la SEQ ID n°2 et conférant une résistance aux inhibiteurs de l'AHA synthase.

3. Méthode de transformation selon l'une quelconque des revendications 1 ou 2, dans laquelle on utilise un herbicide de type imidazolinone pour la sélection.

4. Méthode de transformation selon la revendication 3, dans laquelle on utilise l'acide (RS)-2-(4-isopropyl-4-méthyl-5-oxo-2-imidazolin-2-yl)-5-méthoxy-méthylnicotinique pour la sélection.

5. Plante de pomme de terre transgénique produite par la méthode de transformation selon l'une quelconque des revendications 1 à 4, dans laquelle la plante présente une résistance élevée aux herbicides de type imidazolinone.

6. Plante de pomme de terre transgénique selon la revendication 5, dans laquelle la plante présente une résistance élevée à l'acide (RS)-2-(4-isopropyl-4-méthyl-5-oxo-2-imidazolin-2-yl)-5-méthoxyméthyl-nicotinique.

7. Cellule d'une plante de pomme de terre transgénique selon la revendication 5 ou 6.

8. Tubercule d'une plante de pomme de terre transgénique selon la revendication 5 ou 6.

9. Utilisation d'un vecteur d'expression tel que **caractérisé** selon la revendication 1 ou 2, pour la sélection de cellules de plante de pomme de terre transgénique, de cultures de tissus de pommes de terre transgéniques ou de plantes de pomme de terre transgéniques.


### SEQUENCE LISTING

<110> BASF Plant Science GmbH
<120> Use of AHAS mutant genes as selection marker in potato transformation
<130> NAE387-02
<140> 0093-53700
   <141> 2002-07-08
<160> 2
<170> PatentIn Ver. 2.1
   <210> 1
   <211> 5717
   <212> DNA
   <213> Arabidopsis thaliana
<220>
   <221> CDS
   <222> (2484)..(4493)
<400> 1
   tctagattat gtatttccaa ctttcattaa caatataatc gcatataaat gaaaaatcgt 60
   ttccaggata atattttgat gaaatctcat attattgttc gtactcggat tgatgttgaa 120
   ggcttgaagc gcttcaaatt atagaccaga ttatttaagt ttttcttttg tttactccat 180
   atcaatttga tccattatac tacctaagaa aatttaggta acatagaatt atttattgtt 240
   atagtaaaaa aaaggaaaac cacaaaaata atctactttt acgtatatac tattttcatg 300
   acataagtaa ttaagttgta caactttttt ttaatgaaaa gagagagtaa atttatcatg 360
   ttcatgtgta gttacctcgt gaataaccga cggttatata gacgcctaac atgaattgtt 420
   cagttgaaga cagttcaaaa catgtgtttc actctaaaat cctcaacaaa aaaaaagtgt 480
   taaaatttgt aaacctcttt caagcaaaaa aagaaaaagt gttagaatcc caagattctt 540
   tcataatccg gaatcttggc tgaaaacgta taaaagagat tgacgtagta acaaggagtc 600
   ttggtatgct tccatgcttt ttatcctttt ttgtcatgga accatgattt ggttaccatt 660
   tattatgtaa ccgaaatttt cattgtaata atgaatattt aaatttttag caaaaaaaaa 720
   caaaaaaaaa caaggagtct tgtcttcgtt ctcaaatttc agagctcttg cacttttcaa 780
   gagttttact ttgatgagtg agacatttgt ctttttagtg tttattttct aaacttaaaa 840
   tagtagcatc aacatcactc aattataatt cttaagatgt tgtagaaaaa tattttatag 900
   atggaaagta atcgatatta agacaaataa gaaaccaaac cggactttgt gttcagaccg 960
   aatcaaatct gaattggaga aattatggtg gaggcgaaag tcaacggaac taaagtataa 1020
   aaccaaatgt caaaaataaa acccaatttt catccttaaa cgaacctgct gaaaccctaa 1080
   tttcgattac caattccgat ctaaaaagaa gtcatggaag ccattgattc cgcaatcgat 1140
   cctctcagag atttcgctaa gagcagtgtt cgtctcgtcc agcgctgtca caaacccgat 1200
   cgcaagggta acgccttttc tcaaaaaaat ctcatttccg atttttgatc tgtagattag 1260
   ggttttctga aattttgata tcatttgtaa ttgaattggt tatcagaatt cacgaaagta 1320
   gctgtgcgta cggcgattgg atttgtggtg atgggattcg ttggattctt cgtgaagctc 1380
   gttttcatcc caatcaacaa catcatcgtt ggatcttctt agtgtagtac tttctttacg 1440
   aggtaattga tctcgcatta tatatctaca ttttggttat gttacttgac atatagtcat 1500
   tgattcaata gttctgttaa ttcctttaaa gatcattttg actagaccac attcttggtt 1560
   cattcctcaa taatttgtaa tcatattggt ggatatagaa gtagattggt tatagatcag 1620
   atagtggaag actttaggat gaatttcagc tagttttttt ttttggctta ttgtctcaaa 1680
   agattagtgc tttgctgtct ccattgcttc tgctatcgac acgcttctgt ctccttgtat 1740
   ctttattata tctattcgtc ccatgagttt tgtttgttct gtattcgttc gctctggtgt 1800
   catggatgga gtctctgttc catgtttctg taatgcatgt tgggttgttt catgcaagaa 1860
   atgctgagat aaacactcat ttgtgaaagt ttctaaactc tgaatcgcgc tacaggcaat 1920
   gctccgagga gtaggaggag aagaacgaac caaacgacat tatcagccct ttgaggaagc 1980
   tcttagtttt gttattgttt ttgtagccaa attctccatt cttattccat tttcacttat 2040
   ctcttgttcc ttatagacct tataagtttt ttattcatgt atacaaatta tattgtcatc 2100
   aagaagtatc tttaaaatct aaatctcaaa tcaccaggac tatgtttttg tccaattcgt 2160
   ggaaccaact tgcagcttgt atccattctc ttaaccaata aaaaaagaaa gaaagatcaa 2220
   tttgataaat ttctcagcca caaattctac atttaggttt tagcatatcg aaggctcaat 2280
   cacaaataca atagatagac tagagattcc agcgtcacgt gagttttatc tataaataaa 2340
   ggaccaaaaa tcaaatcccg agggcatttt cgtaatccaa cataaaaccc ttaaacttca 2400
   agtctcattt ttaaacaaat catgttcaca agtctcttct tcttctctgt ttctctatct 2460
   cttgctcatc tttctcctga acc atg gcg gcg gca aca aca aca aca aca aca 2513
                             Met Ala Ala Ala Thr Thr Thr Thr Thr Thr
                             1               5                   10
   tct tct tcg atc tcc ttc tcc acc aaa cca tct cct tcc tcc tcc aaa    2561
   Ser Ser Ser Ile Ser Phe Ser Thr Lys Pro Ser Pro Ser Ser Ser Lys
                       15                  20                  25
   tca cca tta cca atc tcc aga ttc tcc ctc cca ttc tcc cta aac ccc      2609
   Ser Pro Leu Pro Ile Ser Arg Phe Ser Leu Pro Phe Ser Leu Asn Pro
                   30                  35                  40
   aac aaa tca tcc tcc tcc tcc cgc cgc cgc ggt atc aaa tcc agc tct      2657
   Asn Lys Ser Ser Ser Ser Ser Arg Arg Arg Gly Ile Lys Ser Ser Ser
               45                  50                  55
   ccc tcc tcc atc tcc gcc gtg ctc aac aca acc acc aat gtc aca acc      2705
   Pro Ser Ser Ile Ser Ala Val Leu Asn Thr Thr Thr Asn Val Thr Thr
           60                  65                  70
   act ccc tct cca acc aaa cct acc aaa ccc gaa aca ttc atc tcc cga      2753
   Thr Pro Ser Pro Thr Lys Pro Thr Lys Pro Glu Thr Phe Ile Ser Arg
   75                  80                  85                  90
   ttc gct cca gat caa ccc cgc aaa ggc gct gat atc ctc gtc gaa gct      2801
   Phe Ala Pro Asp Gln Pro Arg Lys Gly Ala Asp Ile Leu Val Glu Ala
                       95                  100                 105
   tta gaa cgt caa ggc gta gaa acc gta ttc gct tac cct gga ggt gca      2849
   Leu Glu Arg Gln Gly Val Glu Thr Val Phe Ala Tyr Pro Gly Gly Ala
                   110                 115                 120
   tca atg gag att cac caa gcc tta acc cgc tct tcc tca atc cgt aac      2897
   Ser Met Glu Ile His Gln Ala Leu Thr Arg Ser Ser Ser Ile Arg Asn
               125                 130                 135
   gtc ctt cct cgt cac gaa caa gga ggt gta ttc gca gca gaa gga tac      2945
   Val Leu Pro Arg His Glu Gln Gly Gly Val Phe Ala Ala Glu Gly Tyr
           140                 145                 150
   gct cga tcc tca ggt aaa cca ggt atc tgt ata gcc act tca ggt ccc      2993
   Ala Arg Ser Ser Gly Lys Pro Gly Ile Cys Ile Ala Thr Ser Gly Pro
   155                 160                 165                 170
   gga gct aca aat ctc gtt agc gga tta gcc gat gcg ttg tta gat agt      3041
   Gly Ala Thr Asn Leu Val Ser Gly Leu Ala Asp Ala Leu Leu Asp Ser
                       175                 180                 185
   gtt cct ctt gta gca atc aca gga caa gtc cct cgt cgt atg att ggt      3089
   Val Pro Leu Val Ala Ile Thr Gly Gln Val Pro Arg Arg Met Ile Gly
                   190                 195                 200
   aca gat gcg ttt caa gag act ccg att gtt gag gta acg cgt tcg att      3137
   Thr Asp Ala Phe Gln Glu Thr Pro Ile Val Glu Val Thr Arg Ser Ile
               205                 210                 215
   acg aag cat aac tat ctt gtg atg gat gtt gaa gat atc cct agg att      3185
   Thr Lys His Asn Tyr Leu Val Met Asp Val Glu Asp Ile Pro Arg Ile
           220                 225                 230
   att gag gaa gct ttc ttt tta gct act tct ggt aga cct gga cct gtt      3233
   Ile Glu Glu Ala Phe Phe Leu Ala Thr Ser Gly Arg Pro Gly Pro Val
   235                 240                 245                 250
   ttg gtt gat gtt cct aaa gat att caa caa cag ctt gcg att cct aat     3281
   Leu Val Asp Val Pro Lys Asp Ile Gln Gln Gln Leu Ala Ile Pro Asn
                   255                 260                 265
   tgg gaa cag gct atg aga tta cct ggt tat atg tct agg atg cct aaa     3329
   Trp Glu Gln Ala Met Arg Leu Pro Gly Tyr Met Ser Arg Met Pro Lys
               270                 275                 280
   cct ccg gaa gat tct cat ttg gag cag att gtt agg ttg att tct gag     3377
   Pro Pro Glu Asp Ser His Leu Glu Gln Ile Val Arg Leu Ile Ser Glu
           285                 290                 295
   tct aag aag cct gtg ttg tat gtt ggt ggt ggt tgt ttg aat tct agc     3425
   Ser Lys Lys Pro Val Leu Tyr Val Gly Gly Gly Cys Leu Asn Ser Ser
       300                 305                 310
   gat gaa ttg ggt agg ttt gtt gag ctt acg ggg atc cct gtt gcg agt     3473
   Asp Glu Leu Gly Arg Phe Val Glu Leu Thr Gly Ile Pro Val Ala Ser
   315                 320                 325                 330
   acg ttg atg ggg ctg gga tct tat cct tgt gat gat gag ttg tcg tta     3521
   Thr Leu Met Gly Leu Gly Ser Tyr Pro Cys Asp Asp Glu Leu Ser Leu
                   335                 340                 345
   cat atg ctt gga atg cat ggg act gtg tat gca aat tac gct gtg gag     3569
   His Met Leu Gly Met His Gly Thr Val Tyr Ala Asn Tyr Ala Val Glu
               350                 355                 360
   cat agt gat ttg ttg ttg gcg ttt ggg gta agg ttt gat gat cgt gtc     3617
   His Ser Asp Leu Leu Leu Ala Phe Gly Val Arg Phe Asp Asp Arg Val
           365                 370                 375
   acg ggt aag ctt gag gct ttt gct agt agg gct aag att gtt cat att     3665
   Thr Gly Lys Leu Glu Ala Phe Ala Ser Arg Ala Lys Ile Val His Ile
       380                 385                 390
   gat att gac tcg gct gag att ggg aag aat aag act cct cat gtg tct     3713
   Asp Ile Asp Ser Ala Glu Ile Gly Lys Asn Lys Thr Pro His Val Ser
   395                 400                 405                 410
   gtg tgt ggt gat gtt aag ctg gct ttg caa ggg atg aat aag gtt ctt     3761
   Val Cys Gly Asp Val Lys Leu Ala Leu Gln Gly Met Asn Lys Val Leu
                   415                 420                 425
   gag aac cga gcg gag gag ctt aag ctt gat ttt gga gtt tgg agg aat     3809
   Glu Asn Arg Ala Glu Glu Leu Lys Leu Asp Phe Gly Val Trp Arg Asn
               430                 435                 440
   gag ttg aac gta cag aaa cag aag ttt ccg ttg agc ttt aag acg ttt     3857
   Glu Leu Asn Val Gln Lys Gln Lys Phe Pro Leu Ser Phe Lys Thr Phe
           445                 450                 455
   ggg gaa gct att cct cca cag tat gcg att aag gtc ctt gat gag ttg     3905
   Gly Glu Ala Ile Pro Pro Gln Tyr Ala Ile Lys Val Leu Asp Glu Leu
       460                 465                 470
   act gat gga aaa gcc ata ata agt act ggt gtc ggg caa cat caa atg    3953
   Thr Asp Gly Lys Ala Ile Ile Ser Thr Gly Val Gly Gln His Gln Met
   475                 480                 485                 490
   tgg gcg gcg cag ttc tac aat tac aag aaa cca agg cag tgg cta tca    4001
   Trp Ala Ala Gln Phe Tyr Asn Tyr Lys Lys Pro Arg Gln Trp Leu Ser
                   495                 500                 505
   tca gga ggc ctt gga gct atg gga ttt gga ctt cct gct gcg att gga    4049
   Ser Gly Gly Leu Gly Ala Met Gly Phe Gly Leu Pro Ala Ala Ile Gly
               510                 515                 520
   gcg tct gtt gct aac cct gat gcg ata gtt gtg gat att gac gga gat    4097
   Ala Ser Val Ala Asn Pro Asp Ala Ile Val Val Asp Ile Asp Gly Asp
           525                 530                 535
   gga agc ttt ata atg aat gtg caa gag cta gcc act att cgt gta gag    4145
   Gly Ser Phe Ile Met Asn Val Gln Glu Leu Ala Thr Ile Arg Val Glu
       540                 545                 550
   aat ctt cca gtg aag gta ctt tta tta aac aac cag cat ctt ggc atg    4193
   Asn Leu Pro Val Lys Val Leu Leu Leu Asn Asn Gln His Leu Gly Met
   555                 560                 565                 570
   gtt atg caa tgg gaa gat cgg ttc tac aaa gct aac cga gct cac aca    4241
   Val Met Gln Trp Glu Asp Arg Phe Tyr Lys Ala Asn Arg Ala His Thr
                   575                 580                 585
   ttt ctc ggg gat ccg gct cag gag gac gag ata ttc ccg aac atg ttg    4289
   Phe Leu Gly Asp Pro Ala Gln Glu Asp Glu Ile Phe Pro Asn Met Leu
               590                 595                 600
   ctg ttt gca gca gct tgc ggg att cca gcg gcg agg gtg aca aag aaa    4337
   Leu Phe Ala Ala Ala Cys Gly Ile Pro Ala Ala Arg Val Thr Lys Lys
           605                 610                 615
   gca gat ctc cga gaa gct att cag aca atg ctg gat aca cca gga cct    4385
   Ala Asp Leu Arg Glu Ala Ile Gln Thr Met Leu Asp Thr Pro Gly Pro
       620                 625                 630
   tac ctg ttg gat gtg att tgt ccg cac caa gaa cat gtg ttg ccg atg    4433
   Tyr Leu Leu Asp Val Ile Cys Pro His Gln Glu His Val Leu Pro Met
   635                 640                 645                 650
   atc ccg aat ggt ggc act ttc aac gat gtc ata acg gaa gga gat ggc    4481
   Ile Pro Asn Gly Gly Thr Phe Asn Asp Val Ile Thr Glu Gly Asp Gly
                   655                 660                 665
   cgg att aaa tac tgagagatga aaccggtgat tatcagaacc ttttatggtc        4533
   Arg Ile Lys Tyr
               670
   tttgtatgca tatggtaaaa aaacttagtt tgcaatttcc tgtttgtttt ggtaatttga 4593
   gtttctttta gttgttgatc tgcctgcttt ttggtttacg tcagactact actgctgttg 4653
   ttgtttggtt tcctttcttt cattttataa ataaataatc cggttcggtt tactccttgt 4713
   gactggctca gtttggttat tgcgaaatgc gaatggtaaa ttgagtaatt gaaattcgtt 4773
   attagggttc taagctgttt taacagtcac tgggttaata tctctcgaat cttgcatgga 4833
   aaatgctctt accattggtt tttaattgaa atgtgctcat atgggccgtg gtttccaaat 4893
   taaataaaac tacgatgtca tcgagaagta aaatcaactg tgtccacatt atcagttttg 4953
   tgtatacgat gaaatagggt aattcaaaat ctagcttgat atgccttttg gttcatttta 5013
   accttctgta aacatttttt cagattttga acaagtaaat ccaaaaaaaa aaaaaaaaaa 5073
   tctcaactca acactaaatt attttaatgt ataaaagatg cttaaaacat ttggcttaaa 5133
   agaaagaagc taaaaacata gagaactctt gtaaattgaa gtatgaaaat atactgaatt 5193
   gggtattata tgaatttttc tgatttagga ttcacatgat ccaaaaagga aatccagaag 5253
   cactaatcag acattggaag taggaatatt tcaaaaagtt tttttttttt aagtaagtga 5313
   caaaagcttt taaaaaatag aaaagaaact agtattaaag ttgtaaattt aataaacaaa 5373
   agaaattttt tatatttttt catttctttt tccagcatga ggttatgatg gcaggatgtg 5433
   gatttcattt ttttcctttt gatagccttt taattgatct attataattg acgaaaaaat 5493
   attagttaat tatagatata ttttaggtag tattagcaat ttacacttcc aaaagactat 5553
   gtaagttgta aatatgatgc gttgatctct tcatcattca atggttagtc aaaaaaataa 5613
   aagcttaact agtaaactaa agtagtcaaa aattgtactt tagtttaaaa tattacatga 5673
   ataatccaaa acgacattta tgtgaaacaa aaacaatatc taga 5717
<210> 2
   <211> 670
   <212> PRT
   <213> Arabidopsis thaliana
<400> 2
   Met Ala Ala Ala Thr Thr Thr Thr Thr Thr Ser Ser Ser Ile Ser Phe
   1               5                   10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
       130                 135                 140
   Gln Gly Gly Val Phe Ala Ala Glu Gly Tyr Ala Arg Ser Ser Gly Lys
   145                 150                 155                 160
   Pro Gly Ile Cys Ile Ala Thr Ser Gly Pro Gly Ala Thr Asn Leu Val
                   165                 170                 175
   Ser Gly Leu Ala Asp Ala Leu Leu Asp Ser Val Pro Leu Val Ala Ile
               180                 185                 190
   Thr Gly Gln Val Pro Arg Arg Met Ile Gly Thr Asp Ala Phe Gln Glu
           195                 200                 205
   Thr Pro Ile Val Glu Val Thr Arg Ser Ile Thr Lys His Asn Tyr Leu
       210                 215                 220
   Val Met Asp Val Glu Asp Ile Pro Arg Ile Ile Glu Glu Ala Phe Phe
   225                 230                 235                 240
   Leu Ala Thr Ser Gly Arg Pro Gly Pro Val Leu Val Asp Val Pro Lys
                   245                 250                 255
   Asp Ile Gln Gln Gln Leu Ala Ile Pro Asn Trp Glu Gln Ala Met Arg
               260                 265                 270
   Leu Pro Gly Tyr Met Ser Arg Met Pro Lys Pro Pro Glu Asp Ser His
           275                 280                 285
   Leu Glu Gln Ile Val Arg Leu Ile Ser Glu Ser Lys Lys Pro Val Leu
       290                 295                 300
   Tyr Val Gly Gly Gly Cys Leu Asn Ser Ser Asp Glu Leu Gly Arg Phe
   305                 310                 315                 320
   Val Glu Leu Thr Gly Ile Pro Val Ala Ser Thr Leu Met Gly Leu Gly
                   325                 330                 335
   Ser Tyr Pro Cys Asp Asp Glu Leu Ser Leu His Met Leu Gly Met His
               340                 345                 350
   Gly Thr Val Tyr Ala Asn Tyr Ala Val Glu His Ser Asp Leu Leu Leu
           355                 360                 365
   Ala Phe Gly Val Arg Phe Asp Asp Arg Val Thr Gly Lys Leu Glu Ala
       370                 375                 380
   Phe Ala Ser Arg Ala Lys Ile Val His Ile Asp Ile Asp Ser Ala Glu
   385 390 395 400
   Ile Gly Lys Asn Lys Thr Pro His Val Ser Val Cys Gly Asp Val Lys
   405 410 415
   Leu Ala Leu Gln Gly Met Asn Lys Val Leu Glu Asn Arg Ala Glu Glu
   420 425 430
   Leu Lys Leu Asp Phe Gly Val Trp Arg Asn Glu Leu Asn Val Gln Lys
   435 440 445
   Gln Lys Phe Pro Leu Ser Phe Lys Thr Phe Gly Glu Ala Ile Pro Pro
   450 455 460
   Gln Tyr Ala Ile Lys Val Leu Asp Glu Leu Thr Asp Gly Lys Ala Ile
   465 470 475 480
   Ile Ser Thr Gly Val Gly Gln His Gln Met Trp Ala Ala Gln Phe Tyr
   485 490 495
   Asn Tyr Lys Lys Pro Arg Gln Trp Leu Ser Ser Gly Gly Leu Gly Ala
   500 505 510
   Met Gly Phe Gly Leu Pro Ala Ala Ile Gly Ala Ser Val Ala Asn Pro
   515 520 525
   Asp Ala Ile Val Val Asp Ile Asp Gly Asp Gly Ser Phe Ile Met Asn
   530 535 540
   Val Gln Glu Leu Ala Thr Ile Arg Val Glu Asn Leu Pro Val Lys Val
   545 550 555 560
   Leu Leu Leu Asn Asn Gln His Leu Gly Met Val Met Gln Trp Glu Asp
   565 570 575
   Arg Phe Tyr Lys Ala Asn Arg Ala His Thr Phe Leu Gly Asp Pro Ala
   580 585 590
   Gln Glu Asp Glu Ile Phe Pro Asn Met Leu Leu Phe Ala Ala Ala Cys
   595 600 605
   Gly Ile Pro Ala Ala Arg Val Thr Lys Lys Ala Asp Leu Arg Glu Ala
   610 615 620
   Ile Gln Thr Met Leu Asp Thr Pro Gly Pro Tyr Leu Leu Asp Val Ile
   625 630 635 640
   Cys Pro His Gln Glu His Val Leu Pro Met Ile Pro Asn Gly Gly Thr
   645 650 655
   Phe Asn Asp Val Ile Thr Glu Gly Asp Gly Arg Ile Lys Tyr
   660 665 670